# EUROPEAN PATENT SPECIFICATION

(11) **EP 0 366 820 B1**
(45) Date of publication and mention of the grant of the patent: **04.05.1994**
(21) Application number: 88118232.3
(22) Date of filing: 02.11.1988
(51) Int. Cl.: G01N 33/68

(54) **Method of primary diagnosis for sick animals and diagnostic reagent for such method**
Verfahren zur Anfangsdiagnose bei kranken Tieren und diagnostisches Reagens für dieses Verfahren
Méthode de diagnostic primaire pour les animaux malades et réactif diagnostique à cet effet

(43) Date of publication of application: 09.05.1990
(73) Proprietor: KABUSHIKI KAISHA SAIKIN KAGAKU KENKYUJO, Sendai-shi Miyagi-ken (JP)
(72) Inventor: Tamura, Keiji, Sendai-shi Kiyagi-ken (JP)
(74) Representative: LOUIS, PÖHLAU, LOHRENTZ & SEGETH

(56) References cited:
- EP-A- 0 199 196
- US-A- 4 492 753
- CHEMICAL ABSTRACTS, vol. 92, no. 9, March 1980, Columbus, OH (US); R.-S. TAN et al., p. 292, no. 72114n#
- CHEMICAL ABSTRACTS, vol. 98, no. 17, April 1983, Columbus, OH (US); T.O. KLEINE et al., p. 281, no. 139908u#
- CHEMICAL ABSTRACTS, vol. 99, no. 3, July 1983, Columbus, OH (US); K. KASUGA, p. 334, no. 19228w#
- CHEMICAL ABSTRACTS, vol. 99, no. 1, July 1983, Columbus, OH (US); Y. SHIBATA et al., p. 386, no. 4005x#

## Description

The present invention relates to a method of primary diagnosis for diagnosing whether a breeding animal, pet animal or experiment animal is sick or not. It also relates to a reagent for use in such a method.

It is commonly practiced that cattle, pigs, horses, goats, sheep, domestic fowls, etc. are raised for stockbreeding and dogs, cats, etc. are kept as pet animals. Also, rabbits, guinea pigs, mice, dogs, cats, rats, etc. are used as animals for experimental purposes.

In case of stockbreeding, it is necessary to exclude sick animals from production line so as to keep the quality of meat, milk, eggs, etc. at a certain level. There has been, however, no effective means to identify the sick animals hitherto, and breeders are usually trying to detect them according to their own experience and then asking veterinarians for diagnosis.

Judgment based on such experience is, however, difficult to make in many cases unless conditions of the disease is more advanced, which often results in delay of treatment.

When young pigs or calves are introduced, they often die quickly because they cannot adapt themselves to the new environment due to some diseases.

Dogs and cats are increasingly kept as pet animals in recent years, but not many people have full knowledge or understanding about their health conditions, and the treatment for the disease of these animals is delayed in many cases.

Experiment animals are judged of their health conditions by changes of body weight, and only those regarded as normal are offered for experimental purposes.

However, many of these animals die from unknown causes after the starting of the experiment. The responses of these animals to pharmaceutical substances are abnormally high or abnormally low in some cases, and it is very likely that they have some kind of diseases.

The object of the present invention is to offer a method of primary diagnosis on health conditions of breeding animals, pet animals and experiment animals and to identify and exclude those sick animals.

This object is achieved by a method according to claim 1 and the reagent according to claim 6. Preferred embodiments are defined in the sub-claims.

Methods for determining α₁-acid glycoprotein are known in the art and are, for example, disclosed in

| | |
|---|---|
| Chemical Abstracts, | **92** (1980):72114n; |
| " | **99** (1983):19228w; |
| " | **99** (1983):4005x; |
| " | **98** (1983):139908u; |
| EP-A-199 196; and | |
| US-A-4 492 753. | |

The present invention will become more apparent from the following description of some embodiments thereof taken in conjunction with the accompanying drawings.
Fig. 1 is a diagram showing the range of α₁-acid glycoprotein values in bovine serum determined by the diagnostic reagent according to the present invention; and
Fig. 2 gives fluctuation of α₁-acid glycoprotein in serum of BCG-infected mice measured by the diagnostic reagent according to the present invention.

The present invention offers a primary diagnostic method for sick animals wherein α₁-acid glycoprotein in body fluids of breeding animals, pet animals and experiment animals is measured, and a reagent for primary diagnosis for sick animals wherein holes for receiving specimen are provided on a diffusion plate containing specific antibody of different kind of animal to α₁-acid glycoprotein of the breeding animals, pet animals and experiment animals. In the manufacture of said reagent, serum of breeding animals, pet animals and experiment animals are treated by ammonium sulfate fractionation, purified by ion-exchange column chromatography, and further purified through gel filtration, α₁-acid glycoprotein thus obtained is given to the different kind of animals to immunize them, and specific antiserum obtained is poured into a diffusion plate.

Here, the breeding animals include cattle, pigs, horses, goats, sheep, domestic fowls, etc., and the pet animals comprises dogs, cats, etc. As the experiment animals, there are rabbits, guinea pigs, rats, mice, dogs, cats, etc.

The body fluids used as detection specimens are: blood, plasma, serum, urine, milk, cerebrospinal fluid, articular fluid, ascites, thoracic fluid, tear, bile, seminal fluid, snivel, saliva, etc.

α₁-acid glycoprotein of the above-mentioned animals is obtainable as follows:
① Primary purification by ammonium sulfate fractionation
   Ammonium sulfate is added to animal serum until 60% saturation is attained. After leaving it at room temperature for 3 hours, the precipitate is removed. Ammonium sulfate is further added to the supernatant to attain 90% saturation. After leaving it at 4°C for 16 hours, α₁-acid glycoprotein is obtained as the precipitate.
② Secondary purification by DEAE-column chromatography
   The fraction of α₁-acid glycoprotein obtained by ammonium sulfate fractionation is dialyzed with 0.02M acetate buffer solution (pH 4.3) and is passed through DEAE-cellulose column equilibrated with the same buffer solution. When elution is performed stepwise by changing the concentration of buffer solution, e.g. when eluted with 0.1M, 0.5M or 2.0M buffer solution, α₁-acid glycoprotein is obtained as fraction eluted with 0.5M buffer solution.
③ Tertiary purification by CM-column chromatoghraphy
   The fraction of α₁-acid glycoprotein purified through DEAE-cellulose column is dialyzed with 0.02M acetate buffer solution (pH 4.3) and is passed through CM-cellulose column equilibrated with the same buffer solution. α₁-acid glycoprotein is obtained as fraction eluted without being adsorbed. After dialyzed with purified water, it is freeze-dried.
④ Quaternary purification by gel filtration
   The fraction of α₁-acid glycoprotein purified with CM-cellulose column is separated according to molecular weight by the column with Sephacryl™ gel. The fraction of α₁-acid glycoprotein is eluted at the molecular weight of about 50,000. After dialysed with purified water, it is freeze-dried to obtain white powder of α₁-acid glycoprotein.

Physicochemical properties of α₁-acid glycoprotein in sera of various types of animals, obtained through the above-mentioned purification procedures, is analyzed, using SDS-polyacrylamide gel electrophoresis, amino acid analyzer, gas chromatography, plate electrofocusing, etc. The results are shown in Table 1.

**Table 1**

| physicochemical properties of α₁-acid glycoprotein in sera of various animals | | | |
|---|---|---|---|
| animal | molecular weight | carbohydrate content | isoelectric point |
| cattle | 38000-49000 | 26.5-47.5 | 3.0-3.8 |
| pig | 47000-50000 | 25.8-41.8 | 3.1-3.8 |
| horse | 42000-46000 | 24.6-38.2 | 3.0-3.7 |
| domestic fowl | 40000-47000 | 21.3-43.8 | - |
| dog | 31000-43000 | 22.8-43.4 | 3.0-3.9 |
| cat | 32000-42000 | 21.5-40.8 | 2.8-3.6 |
| mouse | 40000-45000 | 25.0-42.3 | 2.8-3.6 |

Freund's complete adjuvant is added to α₁-acid glycoprotein of various animals purified as described above to emulsify and this is given subcutaneously to goats, rabbits, sheep, horses, cattle, etc. (animals other than the animal under study). Injection is performed repeatedly every two weeks, and blood is collected after confirming increased antibody value. The blood thus collected is centrifuged and the supernatant is taken to obtain specific antiserum.

With this specific antiserum, α₁-acid glycoprotein can be detected and determined quantitatively by various immunological antigen-antibody reaction. For example, the antigen-antibody reaction with α₁-acid glycoprotein in the specimen is induced by single radial immunodiffusion, double immunodiffusion, immunoelectrophoresis, antigen-antibody crossed electrophoresis, counter-immunoelectrophoresis, rocket electrophoresis, sensitized hemagglutination reaction, passive hemagglutination inhibition reaction, sensitized latex agglutination reaction, turbidimetric immunoassay, radioimmunoassay, enzyme immunoassay, etc. Thus, α₁-acid glycoprotein in blood or body fluid is measured.
(i) Single radial immunodiffusion (SRID method)
   5 µ l of the specimen of serum (antigen) and the like is injected into small holes of agar plate containing anti-α₁-acid glycoprotein. When reaction is performed at room temperature or at 37°C for 24 - 48 hours, α₁-acid glycoprotein in the specimen is diffused radially, reacts with antibody and forms sedimentation ring. Since the antigen concentration and the area of precipitin ring are related with each other linearly, the quantity of α₁-acid glycoprotein in the specimen can be calculated by calibration curve of α₁-acid glycoprotein standard solution. This method is easy to perform, does not require expensive devices and instruments and has high reproducibility. But, it requires relatively long time before final evaluation. Minimum sensitivity is 5 µ g/ml, and the method is suitable for quantitative determination of α₁-acid glycoprotein in serum.
(ii) Passive hemagglutination inhibition reaction (PHA-I method)
   25 µ l each of buffer solution is dispensed into wells of microplates (V-type), and 2ⁿ dilution series are prepared from the specimen by diluter (25 µ l). 25 µ l of anti-α₁-acid glycoprotein serum with a constant titer is added and mixed. After leaving it at room temperature for 30 minutes, it is neutralized. 50 µ l of sensitized erythrocytes (sheep erythrocytes coated with α₁-acid glycoprotein) is added. After mixed and left to stand for more than 2 hours, evaluation is made by hemagglutination. The concentration of α₁-acid glycoprotein is calculated from agglutination value of standard α₁-acid glycoprotein and the dilution multiple of the specimen. This is used for quantitative determination of trace quantity of α₁-acid glycoprotein (10 ng/ml or less) and is convenient for measurement of α₁-acid glycoprotein in urine. But, it is not suitable for quantitative analysis of serum α₁-acid glycoprotein because there is considerable error in 2ⁿ dilution. The sensitivity is 10 ng/ml.
(iii) Enzyme immunoassay (EIA method)
   This is divided into the plate method and the beads method. The specimen and alkaline-phosphatase-labeled α₁-acid glycoprotein are added to the material, on which antibody is coated, and reaction is performed by competitive method at 37°C for 2 hours. After washing, p-nitrophenylphosphate is added; the reaction is stopped after 30 minutes and absorbance is measured at 450 nm. The quantity of α₁-acid glycoprotein is calculated from the standard curve of α₁-acid glycoprotein similarly obtained. The sensitivity is 10 ng/ml.
(iv) Turbidmetric immunoassay (TIA method)
   When 50 µ l of specimen is added to 2.0 ml of anti-α₁-acid glycoprotein serum diluted to a certain titer, agglutination reaction occurs and turbidity results. This turbidity is enhanced with increase of antigen quantity. Reaction is performed at 37°C for 30 minutes and turbidity is measured by spectrophotometer (absorbance at 340 nm). Compensated with specimen blank value, the quantity of α₁-acid glycoprotein is calculated from calibration curve of α₁-acid glycoprotein. When automatic analyzer is used, it can be measured by the reaction at 37°C for 10 minutes with 500 µ l of antibody and 20 µ l of specimen. The method can be quickly performed and has high reproducibility. The sensitivity is 5 µ g/ml.

α₁-acid glycoprotein exists in blood and other body fluids of normal animals such as breeding animals, pet animals and experiment animals and this exhibits high value when the animal is suffering from various diseases, particularly, inflammatory diseases, infection, malignant tumor, etc. The determination of α₁-acid glycoprotein in body fluids by immunological method does not necessarily mean the identification of the disease but may be useful for early detection of the disease. If accompanied with the detailed secondary examination, it will be helpful to actualize the diagnosis with high accuracy.

Since α₁-acid glycoprotein can be purified from various kind of animals, anti-α₁-acid glycoprotein serum with high specificity and high titer can be obtained and the diagnostic reagent with high reliability can be manufactured in easier manner.

### Example 1

A: Purification of bovine α₁-acid glycoprotein
Ammonium sulfate was added to bovine serum to attain 60% saturation. After leaving it at room temperature for 3 hours, precipitate was removed and ammonium sulfate was further added to supernatant until 90% saturation was reached. After leaving the solution at 4°C for 16 hours, α₁-acid glycoprotein was obtained as precipitate.
α₁-acid glycoprotein, obtained by ammonium sulfate fractionation, was dialyzed with 0.02M acetate buffer solution (pH 4.3) and was passed through DEAE-cellulose column equilibrated by the same buffer solution. After washed with 0.1M buffer solution, α₁-acid glycoprotein was obtained as fraction eluted with 0.5M buffer solution.
The fraction of α₁-acid glycoprotein, as purified by DEAE-cellulose column, was dialyzed with 0.02M acetate buffer solution (pH 4.3). It was then passed through CM-cellulose column equilibrated with the same buffer solution. α₁-acid glycoprotein was obtained as unadsorbed eluted fraction. After dialyzed with purified water, it was freeze-dried.
The fraction of α₁-acid glycoprotein, purified with CM-cellulose column was fractionated according to molecular weight by the column with Sephacryl™ gel. The fraction of α₁-acid glycoprotein, eluted at molecular weight of about 50,000 was dialyzed with purified water and was freeze-dried. Thus, highly purified white powder of α₁-acid glycoprotein was obtained.
B: Preparation of specific antiserum to bovine α₁-acid glycoprotein
Physiological saline was added to α₁-acid glycoprotein to attain the concentration of 4 - 20 mg/ml. 0.5 - 10 ml of this solution was mixed with equivalent amount of Freund's complete adjuvant and emulsified and this liquid was injected subcutaneously to the back of rabbit or goat. After 2 weeks, 1 - 20 ml of α₁-acid glycoprotein prepared by similar procedure was further immunized. After 2 more weeks, 1 - 20 ml of emulsified solution of α₁-acid glycoprotein was immunized. On day 10 after the last immunization, blood was collected an serum was separated. After heated at 56°C for 30 minutes and inactivated, sodium azide was added to attain the concentration of 0.05% for the purpose of preservation. Specificity of this antiserum to α₁-acid glycoprotein was measured by Ouchterlony's method using bovine serum as antigen and by immunoelectrophoresis and it was demonstrated that the antibody other than α₁-acid glycoprotein was not contained.
C: Preparation of the reagent for qualitative test of bovine α₁-acid glycoprotein and detection of bovine α₁-acid glycoprotein
Purified agar was added to 1/15M phosphate buffer saline (pH 7.4) to attain the concentration of 1.2%. After heated and dissolved, this was injected into a horizontal container to the thickness of about 1.5 mm and was solidified. The holes with 3 mm diameter were provided with the spacing of about 3 mm each, and antiserum, the serum to be tested and the standard serum containing α₁-acid glycoprotein as obtained according to this invention were applied into each of these holes to fill them up and reaction was performed at room temperature for 24 hours. Thus, the precipitin line between the serum to be tested and antiserum was evaluated. When this precipitin line was observed, it was judged as α₁-acid glycoprotein-positive.
D: Preparation of the reagent for quantitative determination of bovine α₁-acid glycoprotein
Purified agar was added to 1/15M phosphate buffer saline (pH 7.4) to attain the concentration of 1.2%. After heated and dissolved, this was kept at the temperature of 55°C. Further, the antiserum described in the above B was added to attain the final concentration of 2 - 15% and was mixed. This was injected into a horizontal container up to the thickness of about 1.5 mm and was solidified. The holes with 3 mm diameter were provided on it with the spacing of about 15 mm each, and 5 µ l each of original solution of bovine α₁-acid glycoprotein standard solution, 4 x and 16 x diluted solutions and the liquid to be tested were accurately measured and were applied into it. When reaction was performed at room temperature for 24 hours, the diameter of the precipitin ring was found to be in a certain functional relation.
E: Quantitative determination of α₁-acid glycoprotein in normal bovine serum
α₁-acid glycoprotein in normal serum of healthy cows such as Holstein, Japanese Black Cattle, Japanese Shorthorn, Hereford, Jersey, etc. was determined quantitatively by single radial immunodiffusion method using the reagent prepared as described in the above D. The results are as shown in Table 2.

**Table 2**

| α₁-acid glycoprotein in the normal serum of healthy cattle in various kinds | | |
|---|---|---|
| types of cattle | cases | α₁-acid glycoprotein (µg/ml) mean±standard deviation |
| Holstein | 45 | 260±88 |
| Japanese Black Cattle | 40 | 239±40 |
| Japanese Shorthorn | 35 | 263±82 |
| Hereford | 8 | 258±158 |
| Jersey | 15 | 240±98 |

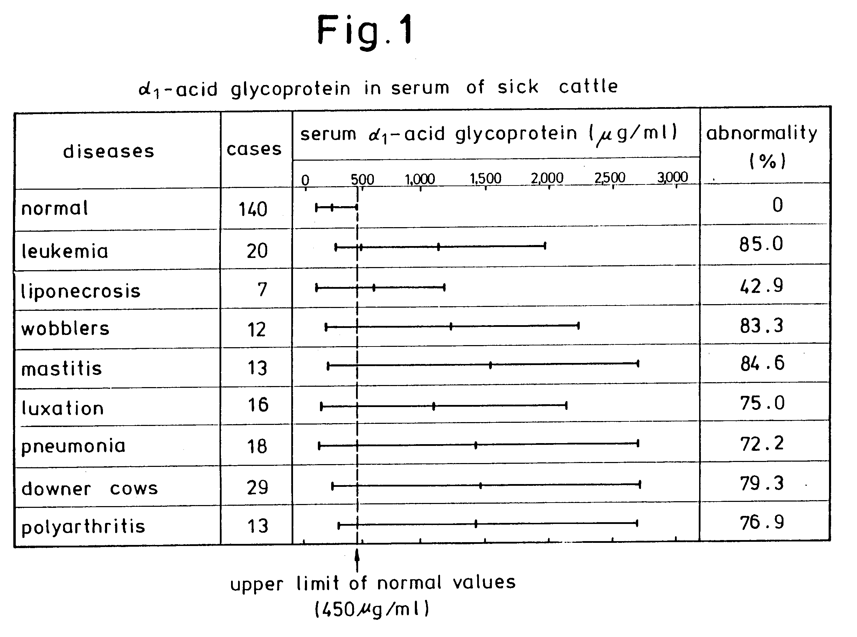

In the values of α₁-acid glycoprotein in the serum of healthy cattle, there was no difference between the types of cattle.
The maximum value (450 µ g/ml) in all of 143 cases was regarded as upper limit for the normal value.
F: Primary diagnosis of various types of sick cattle
For the cattle in healthy condition as well as those diagnosed as having leukemia, mesenteric necrosis, wobblers, mastitis, luxation, pneumonia, downer cows or polyarthritis, α₁-acid glycoprotein in serum was quantitatively determined using the reagent as prepared in the above D. The upper limit of normal values was 450 µ g/ml. The results are as shown in Fig. 1.
The abnormality ratio for normal cattle was 0%, whereas that of sick cattle was: 85.0% for cattle with leukemia, 83.3% for those with wobblers, 84.6% for mastitis, 75.0% for luxation, 72.2% for pneumonia, 79.3% for downer cows, and 76.9% for polyarthritis. Sick cattle were diagnosed as abnormal at higher ratio by primary diagnosis.
Consequently, the diagnostic method according to the present invention offers the information useful for health care of the cattle, for the proper observation of pathological course and for the judgement of prognosis.

### Example 2

The specific antibody obtained in B of the above Example 1 was coated on insoluble carrier. Cow milk was added to this as the specimen, and marker (purified α₁-acid glycoprotein obtained in A of the Example 1 labeled with alkaline-phosphatase) was added, and competitive reaction was performed at 37°C for 2 hours. After washing, p-nitrophenyl-phosphate was added and the reaction was stopped after 30 minutes and absorbance was measured at 450 nm. From the standard curve of α₁-acid glycoprotein similarly obtained, the quantity of α₁-acid glycoprotein in cow milk was calculated. The results are as shown in Table 3.

**Table 3**

| α₁-acid glycoprotein in cow milk | | |
|---|---|---|
| disease | cases | α₁-acid glycoprotein (µg/ml) |
| mastitis apostematosa | 10 | 10 - 680 |
| gangrenous mastitis | 5 | 10 - 1250 |
| healthy cow milk | 10 | < 8 µg |

### Example 3

By the same procedure as A of the Example 1, α₁-acid glycoprotein of pig was purified, and goat was immunized by the same procedure as B. Thus, specific antiserum was obtained.

By the same procedure as described in the above D, reagent for quantitative determination of α₁-acid glycoprotein was prepared, and the quantity of α₁-acid glycoprotein was determined in the serum of healthy Landrace pigs and White York pigs as well as pigs diagnosed as having the diseases as shown in Table 4. The results are as shown in Table 4.

**Table 4**

| α₁-acid glycoprotein in serum of pigs | | | |
|---|---|---|---|
| disease | cases | α₁-acid glycoprotein (µg/ml) | abnormality (%) |
| swine dysentery | 2 | 3260, 4485 | 100 |
| exudative dermatitis | 3 | 1880, 2175, 3600 | 100 |
| miscarrying mother pigs | 3 | 480, 520, 870 | 33 |
| multiple abscess | 4 | 1200, 1260, 1580, 1620 | 100 |
| pneumonia | 5 | 680, 1125, 1300, 1425, 1580 | 100 |
| meningitis | 8 | 720, 885, 890, 900, 1080, 1260, 1580, 2050 | 100 |
| colibacillemia | 15 | 285, 295, 300, 885, 905, 1000, 1085, 1090, 1255, 1320, 1320, 1425, 1550, 1915, 2280 | 80 |
| healthy Landrace pigs | 10 | 295, 450, 395, 415, 395, 415, 325, 505, 250, 450 | 0 |
| healthy White York pigs | 10 | 295, 435, 415, 415, 435, 360, 500, 280, 435, 360 | 0 |
| The value of α₁-acid glycoprotein more than 506 µ g/ml was judged as abnormal. | | | |

When the limit of normal value is set to 505 µ g/ml, which is the maximum value for α₁-acid glycoprotein for healthy Landrace and White York pigs, the pigs with swine dysentery, exudative dermatitis, multiple abscess, pneumonia and meningitis were judged as 100% abnormal, even though number of cases tested was not so many, and the pigs with colibacillemia were judged as 80% abnormal.

Consequently, it was proven to be helpful for the early detection of sick pigs, especially those with inflammatory and infectious diseases.

By primary screening of the procedure described for this example, the pigs with diseases can be identified, and overall judgment is achievable by detailed secondary examinations such as clinical examination, bacteriological and biological examinations, histopathological examination, image diagnosis, etc.

When pigs are diagnosed as having diseases, necessary actions according to symptoms can be taken such as segregation, sterilization, preventive injection, administration of drugs, improvement of breeding control, etc.

As described above, the primary diagnostic method according to the present invention can provide adequate observation and prediction of occurrence of deseases through regular inspection, and it is useful for identifying conditions contaminated with diseases and for health care of seed pigs.

### Example 4

By the same procedure as described in the Example 3, the quantity of α₁-acid glycoprotein in serum of 20 introduced pigs was determined by single radial immunodiffusion method. By primary screening, 4 of these pigs were found to have the abnormally high values.

The results of the detailed examination revealed that these pigs with the abnormally high values of α₁-acid glycoprotein had multiple abscess, meningitis, pneumonia or infectious diseases. Adequate treatment was performed on each individual pig, and all of them restored their health conditions.

When young pigs or pork pigs are newly introduced, the pigs themselves are already suffering from some kind of diseases in many cases, and a number of pigs tend to die quickly because they cannot adapt themselves to the new environmental conditions.

Since prices for these newly introduced pigs are increasingly becoming higher, the death of these pigs is a big problem related to the cost and the profit of the stockbreeding industry. By the primary diagnosis according to the present invention, the health condition of the pigs can be checked and their sudden death can be prevented before it actually occurs.

### Example 5

By the same procedure as A of the Example 1, α₁-acid glycoprotein of horses was purified and this was used to immunize rabbits by the same procedure as B. Thus, specific antiserum was obtained.

By the same procedure as D, reagent for quantitative determination of α₁-acid glycoprotein was prepared, and the quantity of α₁-acid glycoprotein in serum of horses was determined. The results are as shown in Table 5.

**Table 5**

| α₁-acid glycoprotein in serum of horses | | | |
|---|---|---|---|
| diseases | cases | α₁-acid glycoprotein (µg/ml) | abnormality (%) |
| pneumonia | 3 | 1355, 1470, 1525 | 100 |
| cardiopathy | 3 | 400, 525, 780 | 67 |
| trauma | 3 | 320, 1435, 1500 | 67 |
| healthy horses | 6 | 235, 245, 360, 385, 400, 450 | 0 |
| The value of α₁-acid glycoprotein more than 451 µ g/ml was judged abnormal. | | | |

### Example 6

By the same procedure as A of the Example 1, α₁-acid glycoprotein of domestic fowls was purified and this was given to rabbits by the same procedure as B to immunize them. Thus, specific antiserum was obtained.

By the same procedure as D, reagent for quantitative determination of α₁-acid glycoprotein of domestic fowls was prepared and the quantity of α₁-acid glycoprotein in serum of domestic fowls was measured. The results are as shown in Table 6.

**Table 6**

| α₁-acid glycoprotein in serum of domestic fowls | | | |
|---|---|---|---|
| diseases | cases | α₁-acid glycoprotein (µg/ml) | abnormality (%) |
| infectious bronchus | 3 | 420, 865, 945 | 67 |
| staphylococcemia | 3 | 785, 920, 1570 | 100 |
| Marek's disease | 5 | 1030, 1560, 1600, 780, 955 | 100 |
| healthy domestic fowls | 7 | 225, 300, 365, 380, 445, 460, 345 | 0 |
| The value of α₁-acid glycoprotein more than 461 µ g/ml was judged abnormal. | | | |

### Example 7

By the same procedure as A of the Example 1, α₁-acid glycoprotein of dogs was purified and this was given to rabbits by the same procedure as B to immunize them. Thus, specific antiserum was obtained.

By the same procedure as D, reagent for quantitative determination of α₁-acid glycoprotein of dogs was prepared and the quantity of α₁-acid glycoprotein in serum of dogs was measured. The results are as shown in Table 7.

**Table 7**

| α₁-acid glycoprotein in serum of dogs | | | |
|---|---|---|---|
| diseases | cases | α₁-acid glycoprotein (µg/ml) | abnormality (%) |
| nephritis | 2 | 420, 890 | 50 |
| infectious hepatitis | 3 | 1020, 1560, 1800 | 100 |
| fracture | 3 | 480, 620, 1260 | 67 |
| pyometra | 4 | 1050, 2450, 2500, 2800 | 100 |
| distemper | 5 | 520, 875, 1020, 1540, 1420 | 100 |
| healthy dogs | 12 | 320, 260, 440, 325, 245, 335, 500, 480, 475, 350, 365, 425 | 0 |
| The value of α₁-acid glycoprotein more than 501 µ g/ml was judged abnormal. | | | |

### Example 8

By the same procedure as A of the Example 1, α₁-acid glycoprotein of cats was purified and this was given to rabbits by the same procedure as B to immunize them. Thus, specific antiserum was obtained.

By the same procedure as D, reagent for quantitative determination of a α₁-acid glycoprotein of cats was prepared and the quantity of α₁-acid glycoprotein in serum of cats was measured. The results are as shown in Table 8.

**Table 8**

| α₁-acid glycoprotein in serum of cats | | | |
|---|---|---|---|
| diseases | cases | α₁-acid glycoprotein (µg/ml) | abnormality (%) |
| leukemia | 2 | 1880, 2050 | 100 |
| suppurative disease | 3 | 420, 1330, 2260 | 67 |
| trauma | 3 | 382, 430, 1420 | 33 |
| purulent pleuritis | 4 | 1120, 1580, 1800, 1850 | 100 |
| panleukopenia | 4 | 885, 1075, 1200, 1580 | 100 |
| healthy cats | 8 | 285, 335, 400, 420, 480 485, 520, 580 | 0 |
| The value of α₁-acid glycoprotein more than 581 µ g/ml was judged abnormal. | | | |

### Example 9

By the same procedure as A of the Example 1, α₁-acid glycoprotein of mice was purified and this was given to rabbits by the same procedure as B to immunize them. Thus, specific antiserum was obtained.

By the same procedure as D, reagent for quantitative determination of α₁-acid glycoprotein of mice was prepared.

After 0.1 ml each of 10⁵ tubercle bacilli (BCG) was given intravenously to 5 mice (BALB/c; 8-weeks old, male), blood was collected at varying time, and α₁-acid glycoprotein in serum was determined by single radial immunodiffusion method using the above reagent.

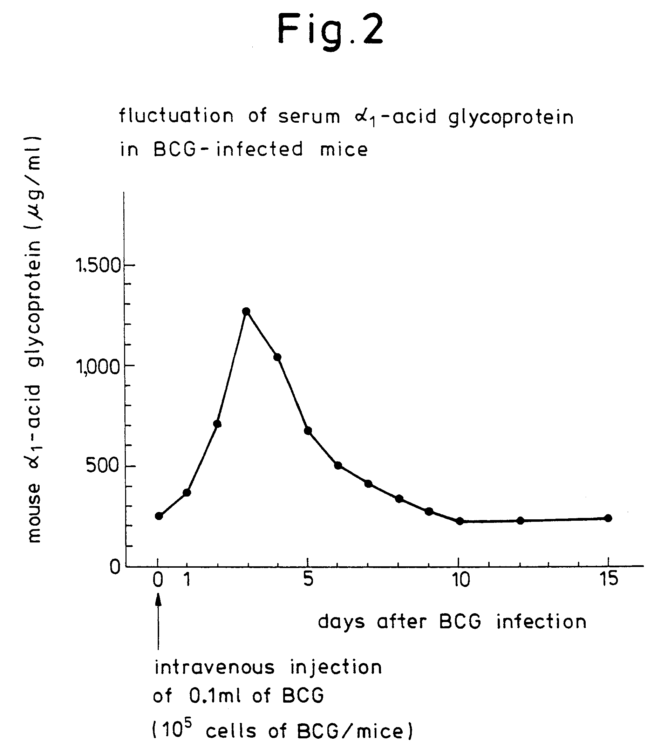

The results are as shown in Fig. 2. After BCG was given, α₁-acid glycoprotein in blood began to increase from 1st day, and the maximum value (1300 µ g/ml) was reached on 3rd day. Then, the value decreased and restored to normal value (240±80 µ g/ml) on 9th day.

As described above, the fluctuation of α₁-acid glycoprotein caused by the infection of BCG was observed. This suggests that this is useful as an index to confirm the biological effects of chemical substances and extracts used for various types of drug efficacy tests.

As explained in the above, the method of primary diagnosis of sick animals based on the present invention makes it possible to primarily diagnose and perform screening for diseases, especially the inflammatory diseases, through the measurement of α₁-acid glycoprotein in body fluids of breeding animals, pet animals and experiment animals. It is useful for early detection of inflammatory diseases, infectious diseases, malignant tumor, etc.

With this method, it is now possible to predict occurrence of diseases through regular inspection.

It is also useful for the identification of the conditions contaminated with diseases, for health care of seed cattle, seed pigs, etc. and also for health control of animals newly introduced.

Further, this would contribute to proper evaluation of effects after therapeutic methods such as chemotherapy, surgical operation, etc. or it would be helpful as marker for judgement of prognosis.

In addition to the effects mentioned above, the diagnostic reagent according to the present invention is useful for simple and easy determination of a large number of specimens. Only small quantity of body fluids such as serum is required for the measurement, and the method has also high reproducibility.

For the manufacture of the diagnostic reagent according to the present invention, it is possible to obtain highly specific anti-animal α₁-acid glycoprotein antibody and to produce the products with high reliability at lower cost and in large quantity.

## Claims

1. A method of primary diagnosis for diagnosing whether a breeding animal, pet animal or experimental animal is sick or not, comprising the steps of
a) fractionating serum of an animal of the same kind as the animal to be diagnosed by using ammonium sulphate as precipitating agent,
b) purifying and extracting the serum fractions obtained by ion exchange column chromatography and gel filtration into highly purified α₁-acid glycoprotein,
c) inoculating said α₁-acid glycoprotein to an animal of a different kind than the animal to be diagnosed, thereby immunising said animal, and obtaining specific antiserum for animals of the kind of the animal to be diagnosed,
d) taking a body fluid obtained from each of a plurality of healthy animals of the kind as the animal to be diagnosed,
e) using said specific antiserum to quantitatively determine α₁-acid glycoprotein in each of said body fluids by immunological antigen-antibody reaction and to define a maximum value as an normal value upper limit,
f) taking a body fluid obtained from the animal to be diagnosed of the same kind as taken from the healthy animals, and
g) quantitatively determining α₁-acid glycoprotein in said body fluid in the same manner as described above, whereby the animal is diagnosed to be sick when a value determined is beyond said upper limit.

2. The method according to claim 1, characterized in that the breeding animals are cattle, pigs, horses, goats, sheep or domestic fowls, the pet animals are dogs or cats and the experiment animals are rabbits, guinea pigs, rats or mice.

3. The method according to claim 1, or 2, characterized in that the body fluid is blood, plasma, serum, urine, milk, cerebrospinal fluid, articular fluid, ascites, thoracic fluid, tear, bile, seminal fluid, snivel or saliva.

4. The method according to one of the preceding claims characterized in that the measurement is made by immunological antigen-antibody reaction.

5. The method according to claim 4, characterized in that the immunological antigen-antibody reaction is single radial immunodiffusion, double immunodiffusion, immunoelectrophoresis, antigen-antibody crossed electrophoresis, counter-immunoelectrophoresis, rocket electrophoresis, sensitized hemagglutination reaction, passive hemagglutination inhibition reaction, sensitized latex agglutination reaction, turbidimetric immunoassay, radioimmunoassay or enzyme immunoassay.

6. A reagent for use in a method of one of the preceding claims, characterized in that holes for application of specimens are furnished on a diffusion plate containing specific antibody of different kind of animals to α₁-acid glycoprotein of breeding animals, pet animal or experiment animals.

## Patentansprüche

1. Verfahren zur Anfangsdiagnose zur Diagnostizierung, ob ein brütendes Tier, ein Haustier oder Versuchstier krank ist oder nicht, umfassend die Stufen:
(a) Fraktionieren des Serums eines Tiers der gleichen Art wie das zu diagnostizierende Tier unter Verwendung von Ammoniumsulfat als Fällungsmittel,
(b) Reinigen und Extrahieren der erhaltenden Serumfraktionen durch Ionenaustausch-Säulenchromatographie und Gelfiltration in hoch gereinigtes α1-Säureglycoprotein
(c) Beimpfen eines Tiers einer von dem zu diagnostizierenden Tier verschiedenen Art mit dem α1-Säureglycoprotein, wodurch das Tier immunisiert wird, und Gewinnen von spezifischem Antiserum für Tiere der Art des zu diagnostizierenden Tiers,
(d) Entnahme von aus jedem einer Vielzahl gesunder Tiere der Art des zu diagnostizierenden Tiers erhaltenen Körperflüssigkeit,
(e) Verwendung des spezifischen Antiserums zur quantitativen Bestimmung von α1-Säureglycoprotein in jeder der Körperflüssigkeiten durch immunologische Antigen-Antikörper-Reaktion und Definieren eines Maximalwerts als obere Grenze des Normalwerts,
(f) Entnahme einer aus dem zu diagnostizierenden Tier gewonnenen Körperflüssigkeit der gleichen Art, wie sie von den gesunden Tieren gewonnen wurde, und
(g) quantitatieve Bestimmung von α1-Säureglycoprotein in der Körperflüssigkeit auf die gleiche Weise wie vorstehend beschrieben, wobei das Tier als krank diagnostiziert wird, wenn der bestimmte Wert oberhalb der oberen Grenze liegt.

2. Verfahren nach Anspruch 1, dadurch gekennzeichnet, daß die brütenden Tiere Rinder, Schweine, Pferde, Ziegen, Schafe oder Haushühner sind, die Haustiere Hunde oder Katzen sind, und die Versuchstiere Kaninchen, Meerschweinchen, Ratten oder Mäuse sind.

3. Verfahren nach Anspruch 1 oder 2, dadurch gekennzeichnet, daß die Körperflüssigkeit Blut, Plasma, Serum, Urin, Milch, cerebrospinale Flüssigkeit, Gelenkflüssigkeit, Ascites, Brustflüssigkeit, Tränen, Galle, Samenflüssigkeit, Nasenflüssigkeit oder Speichelflüssigkeit ist.

4. Verfahren nach einem der vorhergehenden Ansprüche, dadurch gekennzeichnet, daß die Messung durch immunologische Antigen-Antikörper-Reaktion erfolgt.

5. Verfahren nach Anspruch 4, dadurch gekennzeichnet, daß die immunologische Antigen-Antikörper-Reaktion eine einzige Radialimmunodiffusion, eine doppelte Immunodiffusion, Immunoelektrophorese, Antigen-Antikörper-Kreuzelektrophorese, Gegenimmnunoelektrophorese, Rocket-Elektrophorese, sensibilisierte Hemagglutinationsreaktion, passive Hemagglutination-Inhibierungsreaktion, sensibilisierte Latexagglutinatiosreaktion, ein turbidimetrischer Immunoassay, Radioimmunoassay oder Enzymimmunoassay ist.

6. Reagens zur Verwendung in einem Verfahren nach einem der vorhergehenden Ansprüche, dadurch gekennzeichnet, daß an einer Diffusionsplatte, die spezifische Antikörper verschiedener Tierarten gegenüber α1-Säureglycoprotein von brütenden Tieren, Haustieren oder Versuchstieren enthält, Vertiefungen zur Applikation von Proben vorgesehen sind.

## Revendications

1. Méthode pour le diagnostic primaire pour diagnostiquer si un animal d'élevage, un animal de compagnie ou un animal d'expérience est malade ou non, qui comprend les étapes de :
a) fractionnement du sérum d'un animal du même genre que l'animal qui doit faire l'objet d'un diagnostic, en utilisant le sulfate d'ammonium comme agent de précipitation,
b) purification et extraction des fractions de sérum obtenues par chromatographie sur colonne d'échangeurs d'ions et filtration sur gel en une α₁ glycoprotéine acide hautement purifiée,
c) inoculation de cette α₁ glycoprotéine acide à un animal d'un genre différent que l'animal qui doit faire l'objet d'un diagnostic, par là immunisant cet animal et obtenant un anti sérum spécifique pour des animaux du genre des animaux pour lesquels on doit faire un diagnostic,
d) prise d'un liquide corporel obtenu de chacun de la multiplicité des animaux en bonne santé du genre de l'animal pour lequel on doit faire un diagnostic,
e) emploi de cet anti sérum spécifique pour déterminer quantitativement l'α₁ glycoprotéine acide dans chacun des liquides corporels par une réaction immunologique antigène/anticorps et pour définir une valeur maximale en tant que limite supérieure des valeurs normales.
f) prise d'un liquide corporel obtenu d'un animal pour lequel on doit faire un diagnostic du même genre que celui qu'on a pris pour les animaux sains, et
g) détermination quantitative de l'α₁ glycoprotéine acide dans ce liquide corporel de la même façon comme décrit ci-dessus, au moyen de laquelle on peut faire un diagnostic de maladie pour l'animal lorsqu'une valeur déterminée est au-dessus de cette limite supérieure.

2. Méthode selon la revendication 1, caractérisée en ce que les animaux d'élevage sont des bovins, des porcs, des chevaux, des chèvres, des moutons, ou des oiseaux domestiques, les animaux de compagnie sont les chiens et les chats, et les animaux d'expérience sont les lapins, les cobayes, les rats ou les souris.

3. Méthode selon la revendication 1 ou 2, caractérisée en ce que le liquide corporel est le sang, le plasma, le sérum, l'urine, le lait, le liquide céphalorachidien, le liquide articulaire, l'ascite, le fluide thoracique, les larmes, la bile, le fluide séminal, le larmoiement ou la salive.

4. Méthode selon l'une des revendications précédentes, caractérisée en ce que la mesure est effectuée par une réaction immunologique antigène/anticorps.

5. Méthode selon la revendication 4, caractérisée en ce que la réaction immunologique antigène/anticorps est de l'immuno diffusion unique radiale, l'immuno diffusion double, l'immuno électrophorèse, l'électrophorèse croisée antigène/anticorps, l'immuno électrophorèse de comptage, l'électrophorèse fusée, une réaction d'hémagglutination sensibilisée, une reaction d'inhibition passive d'hémagglutination, une réaction d'agglutination sensibilisée du latex, un immuno essai turbidimètrique, un radio immuno essai ou un immuno essai enzymatique.

6. Un réactif pour l'emploi dans une méthode selon l'une des revendications précédentes, caractérisé en ce que des trous pour l'application des échantillons sont fournis sur une plaque de diffusion contenant un anti corps spécifique de différents genres d'animaux pour une α₁ glycoprotéine acide d'animaux d'élevage, d'animaux de compagnie, ou d'animaux d'expérience.
